Europäisches Patentamt

European Patent Office (11) Numéro de publication: **0 040 280**

Office européen des brevets **B1**

(12) **FASCICULE DE BREVET EUROPEEN**

(45) Date de publication du fascicule du brevet: **06.02.85** (51) Int. Cl.⁴: **G 21 F 5/02, A 61 N 5/10**

(21) Numéro de dépôt: **80400662.5**

(22) Date de dépôt: **13.05.80**

(54) **Dispositif de stockage d'une source radioactive et d'irradiation d'un corps par le rayonnement de ladite source.**

(43) Date de publication de la demande:
**25.11.81 Bulletin 81/47**

(45) Mention de la délivrance du brevet:
**06.02.85 Bulletin 85/06**

(84) Etats contractants désignés:
**CH DE FR GB IT LI NL**

(56) Documents cités:
**FR-A-2 331 127**
**FR-A-2 443 122**
**US-A-2 951 162**

(73) Titulaire: **COMMISSARIAT A L'ENERGIE ATOMIQUE Etablissement de Caractère Scientifique Technique et Industriel B.P. 510 F-75752 Paris Cedex 15 (FR)**
(73) Titulaire: **C.G.R. MeV Route de Guyancourt F-78530 Buc (FR)**

(72) Inventeur: **Brethon, Jean-Pierre Résidence Les Pampriers Avenue de Champagne Les Ulis F-91400 Orsay (FR)**
Inventeur: **Guiho, Jean-Paul 8, Place des Pensées F-78320 Le Mesnil Saint Denis (FR)**
Inventeur: **Taniel, Gérard 11, Allée Pasteur F-78690 Les Essarts Le Roi (FR)**
Inventeur: **Viel, Georges 21, rue du Bois du roi F-91400 Orsay (FR)**

(74) Mandataire: **Mongrédien, André et al c/o BREVATOME 25, rue de Ponthieu F-75008 Paris (FR)**

Courier Press, Leamington Spa, England.

## Description

L'invention concerne un dispositif de stockage d'une source radioactive et d'irradiation d'un corps par le rayonnement de cette source, comprenant un corps de protection biologique, un évidement pratiqué dans le corps de protection biologique, un orifice débouchant d'une part sur la paroi extérieure du corps et d'autre part à l'intérieur dudit évidement de ce corps, un disque de protection biologique logé dans l'évidement du corps et mobile en rotation, un logement pratiqué dans le disque et débouchant sur la périphérie de ce disque, une source radioactive contenue dans le logement du disque, des moyens pour déplacer en rotation le disque entre une première position pour laquelle la source est en regard de l'orifice du corps et une deuxième position pour laquelle la source n'est pas en regard de l'orifice.

On connaît des dispositifs du type précité conçus antérieurement à la présente invention qui comportent un élément support d'une source de photons, mobile en translation, et un ensemble optique constitué par une source de lumière, telle qu'une ampoule lumineuse, qui est portée par l'élément-support de la source.

On connaît également, en particulier par le brevet américain US—A—2,951,162, des dispositifs qui comportent une enveloppe métallique procurant une protection biologique contre le rayonnement d'une source radioactive, à l'intérieur de laquelle se trouve un cylindre mobile en rotation autour de son axe longitudinal. Ce cylindre comporte deux trous longitudinaux, diamétralement opposés. La source radioactive est montée sur une barre amovible jouant le rôle de protection biologique et s'engageant dans l'un desdits trous longitudinaux. Une source lumineuse montée sur une barre similaire s'engage dans le deuxième trou longitudinal. L'enveloppe de protection comporte une ouverture conique pour le passage du rayonnement de la source et celui du faisceau lumineux.

De tels dispositifs connus présentent un certain nombre d'inconvénients. En particulier, ils sont d'un poids élevé et de dimensions relativement importantes. Ceci conduit à un balourd gênant dans le cas de certaines applications pour lesquelles le dispositif d'irradiation est animé d'un mouvement de rotation, telles que le traitement thérapeutique d'un patient par irradiation d'une partie de son organisme.

Un autre inconvénient des dispositifs d'irradiation connus est constitué par un fait qu'il est nécessaire d'accéder à l'intérieur de l'appareil pour changer la source lumineuse ou la source radioactive.

Enfin, les appareils d'irradiation de type connu, utilisant des sources radioactives, présentent un autre inconvénient important qui est le changement de ces sources radioactives lorsqu'elles ont perdu au cours du temps une quantité déterminée de leur activité. En particulier, lorsque ces appareils sont utilisés en radiothérapie, il est nécessaire de suspendre leur utilisation et de demander à une équipe spécialisée de procéder sur place au changement de ces sources avec un matériel adapté.

En outre, pour certaines applications telles que le traitement thérapeutique d'un patient, il est nécessaire de délivrer un faisceau de photons de dimensions précises et de régler la position de ce faisceau. Les dispositifs d'irradiation de type connu, qui comportent un système optique éclairant sur un angle solide de 4π stéradians, ne permettent pas une simulation précise du faisceau de photons.

La présente invention a précisément pour objet un dispositif de stockage d'une source de photons et d'irradiation d'un corps par le rayonnement de cette source qui remédie aux inconvénients des dispositifs antérieurement connus.

Un objet de la présente invention est un dispositif d'irradiation destiné à délivrer un faisceau de photons de dimensions précises, facilement réglable, avec un dispositif assurant une première collimation.

Un autre objet de cette invention est de fournir un dispositif permettant le changement des sources radioactives sans avoir recours à des manipulations délicates sur le lieu même de l'utilisation de l'appareil.

Le dispositif d'irradiation selon la présente invention remédie à ces inconvénients, en ce qu'il sert à la fois de dispositif de stockage et de dispositif d'irradiation. La source radioactive est mise en place à l'intérieure du disque de support, son réglage est effectué au moment de la fabrication. Le dispositif muni de sa source déjà réglée est ensuite transporté sur le lieu d'utilisation. Il suffit alors de le monter de façon amovible, par exemple au moyen de vis, sur un bras support, pour qu'il soit prêt à fonctionner sans aucun réglage supplémentaire. Lorsqu'au bout d'un temps déterminé, l'activité de la source radioactive ayant décru, il est devenu nécessaire de la remplacer, le dispositif d'irradiation est démonté de son bras support, et un dispositif d'irradiation identique comportant une source neuve est monté à sa place. On évite ainsi de recourir à une main-d'oeuvre spécialisée, pour procéder au changement de source.

Plus précisement, l'invention a pour objet un dispositif de stockage d'une source radioactive défini par les caractéristiques techniques de la revendication 1.

Ce dispositif comporte notamment les avantages suivants:

— il peut, grâce à sa compacité, être monté aisément sur son support en position opérationnelle, et grâce à la présence d'une protection biologique intégrée se suffisant à elle-même, être directement utilisé pour le transport des sources radioactives, cette protection biologique étant constituée partiellement d'uranium appauvri. Cette fonction de stockage de la source permet d'éviter toutes opérations de chargement ou de déchargement de la source sur le lieu d'utilisation;

— il présente un faible encombrement, tout en assurant une bonne protection biologique et en s'opposant aux fuites de rayonnement directes ou diffuses, grâce à l'utilisation d'un disque rotatif pour la mise en place de la source de photons;

— pendant son utilisation, le dispositif est monté sur un bras-support; il est amovible, et peut être démonté facilement de son bras-support.

L'utilisation d'uranium appauvri, dont la capacité d'absorption du rayonnement est supérieure à celle du plomb, pour réaliser la protection biologique permet d'abaisser le poids de ce dispositif. Ceci permet en particulier de réduire son balourd lorsqu'il est monté sur un bras-support animé d'un mouvement de rotation.

Pour certaines applications, il est nécessaire de délivrer un faisceau lumineux de dimensions précises et facilement réglable servant au repérage du faisceau d'irradiation. Pour de telles applications, le disque de protection biologique du dispositif de stockage selon l'invention présente en outre un passage disposé dans le plan du disque contenant l'axe dudit logement, ce passage débouchant à l'une et à l'autre de ses extrémités dans deux zones de la paroi latérale du disque susceptible d'être simultanément en vis-à-vis desdits premier et deuxième passages;

— au moins une fibre optique disposée dans ledit passage du disque, les extrémités de ladite fibre dites respectivement extrémités réceptrice et émettrice étant maintenues au droit de chacune des extrémités dudit passage par l'intermédiaire de moyens de fixation démontables,

— un dispositif optique solidaire dudit corps pour engendrer un rayonnement lumineux sur l'extrémité réceptrice de ladite fibre ce dispositif étant disposé au droit dudit deuxième passage.

Cette disposition permet une simulation parfaite du faisceau de photons grâce à l'utilisation de l'extrémité émettrice de la fibre optique susceptible d'être définie et positionnée avec précision; elle permet d'autre part, le remplacement aisé du dispositif optique destiné à engendrer le rayonnement lumineux sur l'extrémité réceptrice de la fibre optique.

Le dispositif d'irradiation selon l'invention permet en outre, quand la source est en position de stockage, d'accéder à chacune des extrémités de la fibre optique par le passage du corps au droit duquel le dispositif optique est disposé, ceci tout en limitant à deux, les positions fixes du disque de protection biologique. Ainsi, après démontage du dispositif optique, il est aisé d'introduire ou d'extraire la fibre optique dans le passage court du disque, et de régler son positionnement au droit des extrémités dudit passage.

Selon une disposition préférentielle de l'invention, les axes des extrémités réceptrice et émettrice de la fibre, définissent un angle voisin de 90°. Cette disposition permet de rendre minimale l'irradiation de la fibre optique au cours du fonctionnement de l'appareil.

Selon une autre disposition préférentielle de l'invention, l'angle de rotation du disque de protection lorsque la source passe de la position de stockage à la position d'irradiation, est de 110°. Cette disposition permet de limiter le débit de fuites du rayonnement de la source, lorsque cette dernière est en position de stockage. Elle permet également de limiter la hauteur du corps de protection entourant le disque.

Selon l'invention, le dispositif de stockage d'une source de photons et d'irradiation d'un corps par le rayonnement de cette dernière, comporte de préférence d'une part des moyens électriques de verrouillage du disque dans ladite première position permettant l'irradiation, et d'autre part, des moyens mécaniques pour placer automatiquement le disque dans ladite deuxième position correspondant au stockage de la source.

Par ailleurs, le corps est de préférence constitué par une masse dans laquelle est inclus un bloc en uranium appauvri, ce bloc définissant la portion de la paroi de la cavité située autour de ladite source lorsque ledit disque est dans la position de stockage.

Un avantage important de l'invention est qu'elle permet un remplacement rapide de la source radioactive.

D'autres caractéristiques et avantages de la présente invention apparaîtront plus clairement à la lecture de la description qui suit d'un exemple de réalisation du dispositif de l'invention donnée à titre illustratif en référence aux dessins annexés sur lesquels:

— la figure 1 représente schématiquement un exemple d'installation du dispositif d'irradiation selon l'invention;

— la figure 2 représente une coupe transversale du dispositif de stockage et d'irradiation selon l'invention;

— la figure 3 représente une coupe longitudinale de ce même dispositif;

— la figure 4a représente une vue selon la direction IV de la figure 3;

— la figure 4b représente une vue de détail du dispositif de précollimation;

— les figures 5 et 6 représentent le détail du système de retour en force du disque de protection biologique.

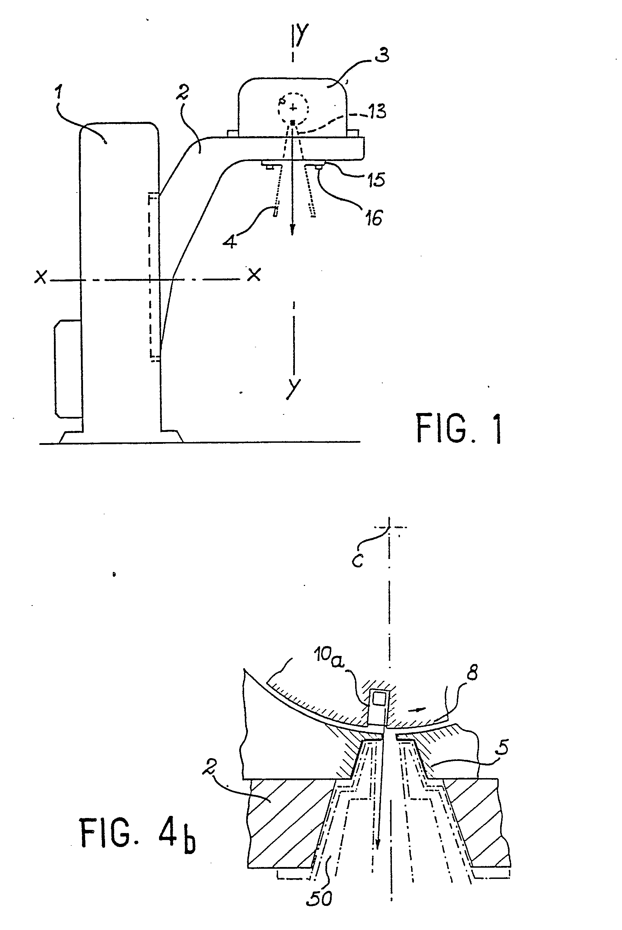

On a représenté sur la figure 1 un dispositif d'irradiation qui peut être utilisé en radiothérapie. Cet appareil comporte un support fixe 1, un bras 2 mobile autour d'un axe X—X, un dispositif d'irradiation monté de façon amovible sur le bras mobile 2, un précollimateur 13 et un dispositif de collimation secondaire 4 d'axe Y—Y. Le précollimateur 13 constitue ledit premier passage ou orifice 5' du corps de protection biologique 7. Le collimateur 4 est fixé de façon amovible sur le bras mobile 2 au moyen d'un plateau support 15, fixé par des vis 16.

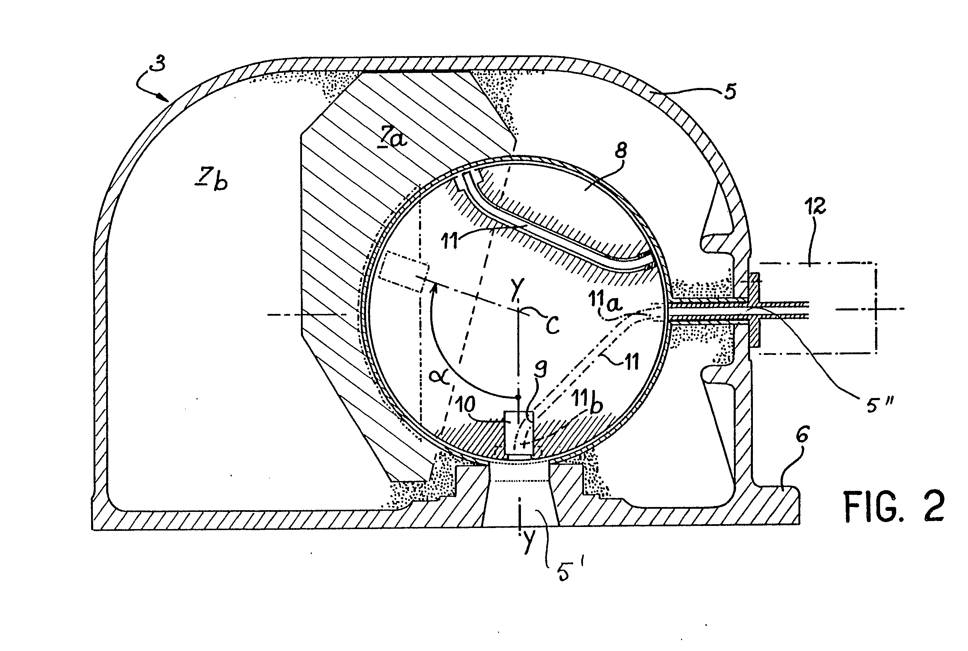

On a représenté sur la figure 2, une coupe transversale du dispositif d'irradiation 3 de la figure 1. Ce dispositif d'irradiation comporte une enceinte 5 ayant la forme d'une cuvette munie d'un rebord 6. Cette enceinte 5 peut être fixée de façon amovible sur le bras 2 au moyen de vis traversant ce rebord 6.

Un blindage fixe 7 de protection biologique est placé à l'intérieur de l'enceinte 5. Le blindage 7 entoure au moins partiellement un disque de protection biologique 8 mobile en rotation autour d'un axe passant par son centre C. Le disque 8 est muni d'un logement 9 destiné à recevoir une source radioactive. Ce logement 9 s'ouvre vers l'extérieur du disque 8 de façon à laisser passer le faisceau de photons délivré par la source radioactive 10. Le blindage fixe 7 se compose d'un bloc d'uranium appauvri 7a, noyé dans un bloc de plomb 7b. Le bloc d'uranium appauvri 7a entoure la source de photons lorsque cette dernière se trouve dans la position de stockage représentée en traits mixtes sur la figure 2. La capacité de l'uranium à absorber le rayonnement étant environ deux fois supérieure à celle du plomb, l'utilisation d'un bloc d'uranium appauvri permet d'assurer une protection biologique satisfaisante, tout en assurant, une réduction du poids et de la taille du dispositif d'irradiation selon l'invention.

Dans l'exemple de réalisation représenté sur la figure 2, une fibre optique 11, destinée à transmettre un flux lumineux, est disposée dans le disque 8. La fibre optique 11 débouche à l'une de ses extrémités 11a, dite extrémité réceptrice, en regard d'une source lumineuse 12 fixée à l'extérieur de l'enceinte 5 et à son autre extrémité 11b, dite extrémité émettrice, suivant l'axe Y—Y. Chacune des extrémités réceptrice et émettrice est maintenue par l'intermédiaire de moyens de réglage de leur positionnement. Le dispositif optique 12 situé en regard dudit deuxième passage 5″ focalise un rayonnement lumineux sur l'extrémité réceptrice de la fibre optique 11, ce dispositif optique 12 pouvant par exemple consister en une source lumineuse associée à un réflecteur pour la focalisation de son faisceau lumineux. Par rayonnement lumineux, il convient d'entendre dans le présent texte un rayonnement électromagnétique situé dans la partie visible du spectre.

Le passage prévu dans le disque 8 de la fibre optique 11 présente une forme sensiblement rectiligne, à l'exception de ses extrémités. De préférence, les extrémités émettrice et réceptrice de la fibre 11 déterminent un angle au centre égal à 90°. Une telle disposition permet de rendre minimale l'irradiation de la fibre optique au cours du fonctionnement de l'appareil. On a désigné sur la figure 2, par α l'angle de rotation du disque 8 qui permet de passer de la position d'irradiation de la source radioactive 10 représentée en traits pleins, à la position de stockage de cette même source, représentée en traits mixtes. La valeur de l'angle α est de 110°. Cette valeur permet de limiter le débit de fuites du rayonnement lorsque la source radioactive 10 est un position de stockage ainsi que la hauteur du corps de protection biologique 7 entourant le disque 8.

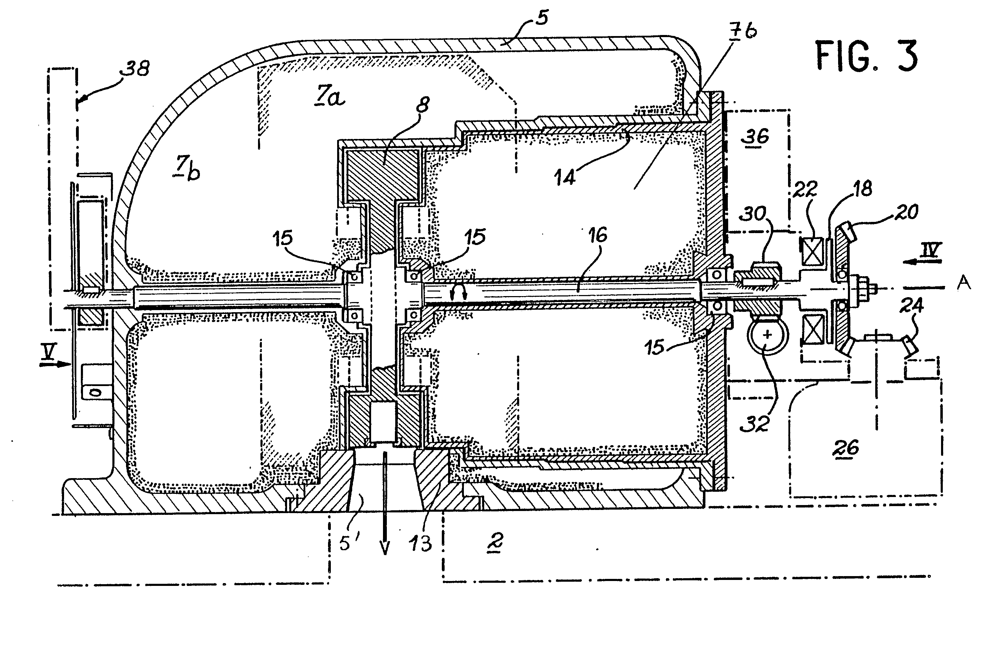

On a représenté sur la figure 3, une coupe longitudinale du dispositif d'irradiation représenté sur la figure 2. Cette vue montre en particulier l'ensemble des positionnements du disque 8 apte à assurer une alternance rapide des positions d'irradiation et de stockage, avec verrouillage du disque en position d'irradiation et retour de ce dernier en position de stockage lors d'une action de sécurité ou dans l'éventualité d'une panne électrique.

Le disque 8, monté sur des roulements 15, est solidaire en rotation de l'arbre 16. Un disque d'embrayage 18 est monté en bout de l'arbre 16. Un pignon 20, monté libre en rotation sur l'arbre 16, peut être accouplé à cet arbre au moyen de l'embrayage électromagnétique 22. Lorsque l'embrayage électromagnétique 22 est alimenté, l'arbre 16 est entraîné en rotation par le pignon 24 du moto-réducteur 26. D'autre part, l'arbre 16 est solidaire en rotation, par exemple au moyen d'un clavetage, d'un pignon 30 entraînant une crémaillère 32 qui est destinée à définir les positions d'irradiation et de stockage du disque 8.

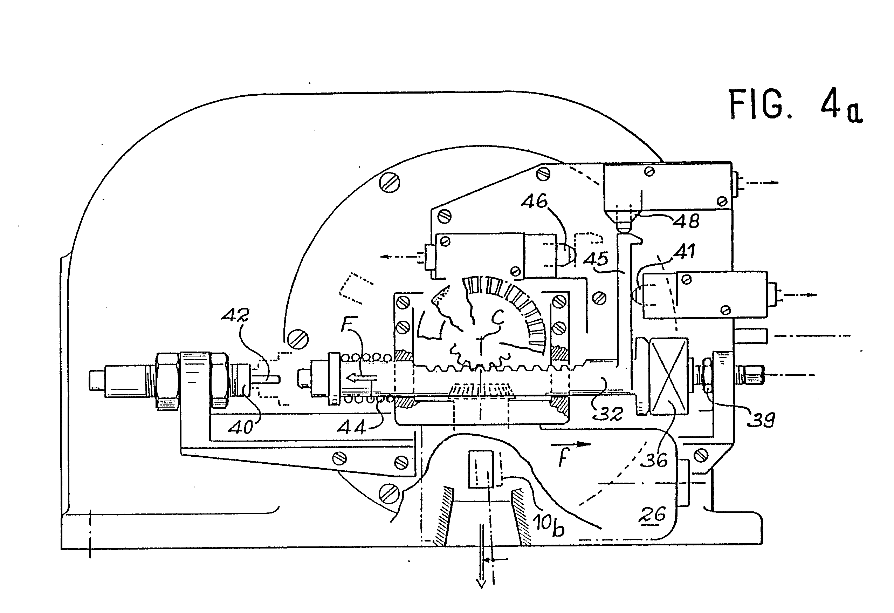

On a d'autre part représenté schématiquement en traits mixtes, le bras mobile 2 sur lequel est monté le dispositif de l'invention, le dispositif de maintien de la position du disque 8 désigné par la référence 36 et enfin, le dispositif 38 de visualisation de la position du disque. Ces dispositifs seront décrits plus en détail en référence aux figures 4, 5 et 6.

On a représenté sur la figure 4a, une vue selon la flèche IV de la figure 3, du dispositif représenté sur cette figure. Cette figure montre en particulier le dispositif 41 de détection de la position du disque 8.

Le crémaillère 32 est susceptible d'être déplacée par un motoréducteur entre une première position, représentée en traits pleins sur la figure 4, pour laquelle elle est en butée contre une ventouse électromagnétique 36 réglable en position au moyen d'un écrou 39, et une deuxième position, représentée en traits mixtes sur la figure 4, pour laquelle la crémaillère 32 se trouve à proximité d'un ensemble amortisseur 40 dont l'action est réglée par le positionnement de la tige 42.

Un contact électrique 41 commande l'arrêt de l'embrayage électromagnétique 22, lorsque la crémaillère 32 parvient, en étant déplacée suivant le sens de la flèche F, à une faible distance de la ventouse électromagnétique 36. Par ailleurs, pour permettre le retour automatique du disque 8 en position de stockage, un ressort 44 est interposé entre une paroi de la crémaillère 32 et une paroi fixe solidaire de l'enceinte 5, de façon à ce que le disque passe automatiquement selon le sens de la flèche F de la position d'irradiation à la position de stockage lors du déclenchement d'un organe de sécurité ou lors d'une panne électrique.

Un deuxième contact électrique 46 actionné par le doigt 45, permet de déterminer la position de fin de course de la crémaillère 32 lorsque celle-ci se troue à proximité du dispositif amortisseur 40.

Enfin, un troisième contact électrique 48 permet de déterminer le début de l'irradiation. En effet, le début de cette irradiation est déterminé par le moment où la source radioactive 10 commence à apparaître dans le précollimateur. Cette position de la source désignée par la référence 10a a été représentée sur la figure 4b.

Enfin, on a représenté, en traits mixtes, sur la figure 4a, une deuxième position 10b de la source radioactive, qui correspond à un léger dépassement par la source radioactive 10 de la position de l'axe Y—Y du dispositif de collimation. Cette disposition a pour but de permettre un rattrapage du jeu inévitable de la crémaillère et du pignon d'entraînement.

On a représenté en 5 sur la figure 4b le dispositif de précollimation avec en outre, représenté en traits mixtes un dispositif de collimation secondaire 50 associé à la tête d'irradiation.

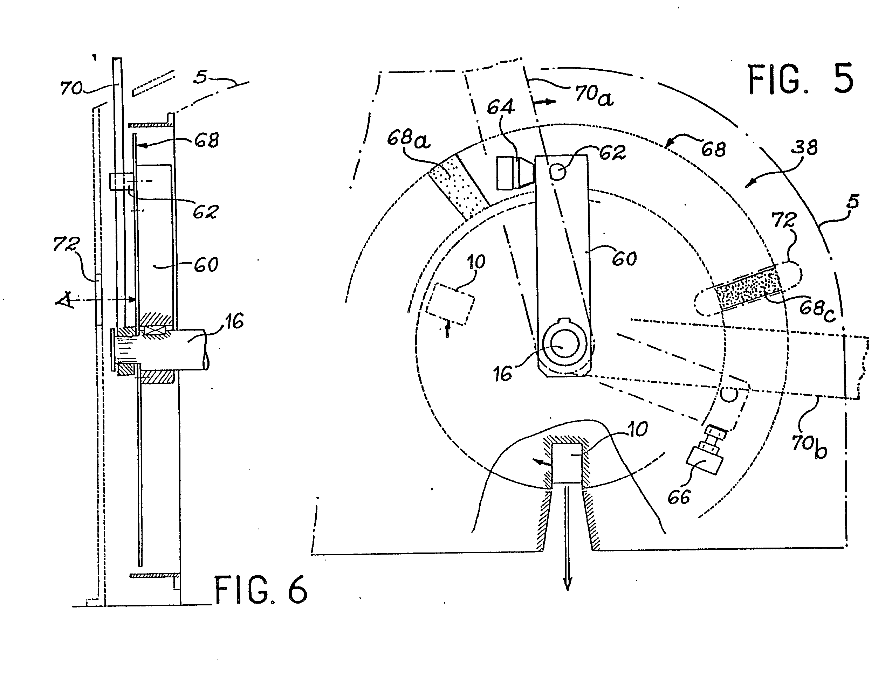

On a représenté sur les figures 5 et 6 le détail du système permettant le retour en force du disque 8 en cas d'incident. Ce dispositif, désigné par la référence générale 38, se compose d'un toc 60 rendu solidaire de l'arbre 16 au moyen d'un clavetage. Le toc 60 porte un doigt de toc 62. La rotation du toc 60 est limitée par deux butées 64 et 66. La butée 64, qui est une butée en caoutchouc, correspond à la position d'irradiation. La butée 66 correspond à la position de stockage de la source radioactive 10. D'autre part, un disque 68 est solidaire du toc 60. Le disque 68 comporte des zones colorées correspondant aux différentes positions du disque. On trouve ainsi successivement une zone 68a de couleur verte correspondant à la position de stockage et une zone 68c rouge correspondant à la position d'irradiation.

Un levier 70, dont le débattement angulaire est limité, est monté fou sur l'extrémité de l'arbre 16. Ce levier permet la commande en force du disque 8 au moyen du toc 60. On remarque que l'arbre 16 ne peut être entraîné grâce à ce dispositif que dans un sens, ce sens correspondant au passage de la position d'irradiation à la position de stockage.

On a représenté sur la figure 5, en traits mixtes, les positions 70a et 70b du levier 70 qui correspondent respectivement aux positions d'irradiation et de stockage. Un indexage extérieur 72 situé en regard du disque 68 permet de déterminer grâce aux zones colorées de ce disque sa position.

Le dispositif d'irradiation qui vient d'être décrit, présente l'avantage important de servir également de chateau de radioprotection pour le transport de la source radioactive. Ceci permet de régler la source en usine, au moment de la fabrication, et d'effectuer d'une manière simple, sans recourir à une main-d'oeuvre spécialisée, le changement de source radioactive. Pour cela, on transporte un dispositif d'irradiation muni d'une source neuve préalablement réglée et prête à l'utilisation sur le lieu de son utilisation, le dispositif d'irradiation jouant le rôle d'un château de radioprotection.

Le dispositif selon l'invention s'applique à la thérapie humaine et vétérinaire, à la métrologie, à la biologie, notamment en recherche, à la médecine et à la recherche vétérinaire.

**Revendications**

1. Dispositif de stockage d'une source radioactive et d'irradiation d'un corps par le rayonnement de cette source, comprenant un corps de protection biologique (7), un évidement pratiqué dans le corps de protection biologique (7), un orifice (5') débouchant d'une part sur la paroi extérieure du corps (7) et d'autre part à l'intérieur dudit évidement de ce corps (7), un disque de protection biologique (8) logé dans l'évidement du corps (7) et mobile en rotation, un logement (9) pratiqué dans le disque (8) et débouchant sur la périphérie de ce disque, une source radioactive (10) contenue dans le logement (9) du disque (8), des moyens pour déplacer en rotation le disque (8) entre une première position pour laquelle la source (10) est en regard de l'orifice (5') du corps (7) et une deuxième position pour laquelle la source (10) n'est pas en regard de l'orifice (5'), caractérisé en ce que le corps de protection biologique (7) comporte un passage (5″) débouchant à l'une de ses extrémités sur la paroi externe du corps (7) et à son autre extrémité dans l'évidement du corps de protection biologique (7), un passage étant pratiqué dans le disque (8), ce passage débouchant à l'une et à l'autre de ses extrémités dans deux zones de la paroi latérale du disque (8) susceptibles d'être simultanément en vis-à-vis dudit orifice (5') et du passage (5″), au moins une fibre optique (11) étant disposée dans le passage du disque (8), les extrémités de la fibre (11) dites respectivement extrémités réceptrice (11a) et émettrice (11b) étant maintenues au droit de chacune des extrémités du passage, un dispositif optique (12) solidaire du corps (7) engendrant un rayon lumineux sur l'extrémité réceptrice (11a) de la fibre (11), ce dispositif étant disposé au droit du passage (5″).

2. Dispositif selon la revendication 1, caractérisé en ce qu'il comporte un précollimateur (13) disposé au droit de l'orifice (5') pratiqué dans le corps de protection biologique (7).

3. Dispositif selon la revendication 2, caractérisé en ce que les axes des extrémités réceptrice et émettrice de la fibre (11) définissent un angle voisin de 90°.

4. Dispositif selon l'une quelconque des revendications 1 à 3, caractérisé en ce qu'il comporte des moyens électriques de verrouillage du disque dans ladite première position.

5. Dispositif selon l'une quelconque des revendications 1 à 4, caractérisé en ce qu'il comprend des moyens mécaniques pour placer automatiquement le disque dans ladite première position.

6. Dispositif selon l'une quelconque des revendications 1 à 5, caractérisé en ce que ledit corps (7) est constitué par une masse de plomb (7b) dans laquelle est inclus un bloc en uranium appauvri (7a), ce bloc définissant la portion de la paroi de la cavité située autour de ladite source lorsque ledit disque est dans ladite deuxième position.

7. Dispositif selon l'une quelconque des revendications 1 à 6, caractérisé en ce que lesdits moyens pour déplacer ledit disque comportent un arbre (16) accouplé, d'une part, à un motoréducteur (26) par l'intermédiaire d'un embrayage électromagnétique (22) et d'autre part, à une crémaillère (32) mobile entre deux positions extrêmes correspondant respectivement auxdites première et deuxième positions du disque (8).

8. Dispositif selon la revendication 7, caractérisé en ce que la position extrême de la crémaillère (32) correspondant à ladite deuxième position du disque (8) est définie par une butée électromagnétique apte à verrouiller la crémaillère, cette butée agissant à l'encontre d'un ressort (64) apte à rappeler la crémaillère dans une position extrême définie par une butée mécanique.

9. Dispositif suivant l'une quelconque des revendications 1 à 8, caractérisé en ce que l'enceinte (5) de la tête d'irradiation (3) comprenant le precollimateur (13) est muni de rebords permettant sa fixation temporaire, au moyen de vis, sur le bras (2) mobile.

## Patentansprüche

1. Vorrichtung zur Lagerung einer radioaktiven Quelle und zur Bestrahlung eines Körpers durch die Strahlung dieser Quelle, mit einem biologischen Schutzkörper (7), einer in dem biologischen Schutzkörper (7) ausgebildeten Aussparung, einer Öffnung (5'), die einerseits an der Außenwand des Körpers (7) und andererseits im Inneren der Aussparung dieses Körpers (7) mündet, einer biologischen Schutzscheibe (8), die in der Aussparung des Körpers (7) angeordnet und drehbewegbar ist, einem Raum (9), der in der Scheibe (8) ausgebildet und zum Umfang dieser Scheibe hin offen ist, einer in dem Raum (9) der Scheibe (8) enthaltenen, radioaktiven Quelle (10), Mitteln zur Drehbewegung der Scheibe (8) zwischen einer ersten Stellung, in der die Quelle (10) der Öffnung (5') des Körpers (7) gegenüberliegt, und einer zweiten Stellung, in der sich die Quelle (10) nicht in Gegenüberlage der Öffnung (5') befindet, dadurch gekennzeichnet, daß der biologische Schutzkörper (7) einen Durchgang (5") umfaßt, der mit einem seiner Enden an der Außenwand des Körpers (7) und mit seinem anderen Ende in der Aussparung des biologischen Schutzkörpers (7) mündet, daß ein Durchgang in der Scheibe (8) ausgebildet ist, daß dieser Durchgang mit seinen beiden Enden in zwei Bereichen der seitlichen Wand der Scheibe (8) mündet, die gleichzeitig in Gegenüberlage der Öffnung (5') und des Durchganges (5") bringbar sind, daß wenigstens eine optische Faser (11) in dem Durchgang der Scheibe (8) angeordnet ist, daß die Enden der Faser (11), welche als Empfangsende (11a) bzw. Emissionsende (11b) bezeichnet werden, auf gerader Linie mit jedem der Enden des Durchganges gehalten sind, und daß eine fest mit dem Körper (7) verbundene, optische Einrichtung (12) ein Lichtstrahlenbündel auf dem Empfangsende (11a) der Faser (11) erzeugt, wobei diese Einrichtung geradlinig zu dem Durchgang (5") angeordnet ist.

2. Vorrichtung nach Anspruch 1, dadurch gekennzeichnet, daß sie einen Vorkollimator (13) umfaßt, der geradlinig zu der in dem biologischen Schutzkörper (7) ausgebildeten Öffnung (5') angeordnet ist.

3. Vorrichtung nach Anspruch 2, dadurch gekennzeichnet, daß die Achsen des Empfangsendes und des Emissionsendes der Faser (11) einen Winkel von etwa 90° begrenzen.

4. Vorrichtung nach einem der Ansprüche 1 bis 3, dadurch gekennzeichnet, daß diese elektrische Mittel zum Verriegeln der Scheibe in der ersten Stellung umfaßt.

5. Vorrichtung nach einem der Ansprüche 1 bis 4, dadurch gekennzeichnet, daß diese mechanische Mittel umfaßt, um die Scheibe automatisch in die erste Stellung zu bringen.

6. Vorrichtung nach einem der Ansprüche 1 bis 5, dadurch gekennzeichnet, daß der Körper (7) von einer Bleimasse (7b) gebildet ist, in der ein Block aus verarmten Uran (7a) eingeschlossen ist, wobei dieser Block den Bereich der Wand des Hohlraumes festlegt, der sich um die Quelle herum befindet, wenn sich die Scheibe in der zweiten Stellung befindet.

7. Vorrichtung nach einem der Ansprüche 1 bis 6, dadurch gekennzeichnet, daß die Mittel zum Verschieben der Scheibe eine Welle (16) umfassen, die einerseits mittels einer elektromagnetischen Kupplung (22) mit einem Getriebemotor (26) und andererseits mit einer Zahnstange (32) gekoppelt ist, die zwischen zwei Endstellungen bewegbar ist, welche der ersten bzw. zweiten Stellung der Scheibe (8) entsprechen.

8. Vorrichtung nach Anspruch 7, dadurch gekennzeichnet, daß die der zweiten Stellung der Scheibe (8) entsprechende Endstellung der Zahnstange (32) durch einen elektromagnetischen Anschlag festgelegt ist, mit dem die Zahnstange verriegelbar ist, und daß dieser Anschlag entgegen einer Feder (64) wirkt, durch die die Zahnstange in eine Endstellung zurückführbar ist, welche durch einen mechanischen Anschlag festgelegt ist.

9. Vorrichtung nach einem der Ansprüche 1 bis 8, dadurch gekennzeichnet, daß das Gehäuse (5) des Bestrahlungskopfes (3), welches den Vorkollimator (13) umfaßt, mit Randleisten versehen ist, die dessen vorübergehende Befestigung mittels Schrauben an dem bewegbaren Arm (2) ermöglichen.

## Claims

1. Device for the storage of a radioactive source and for the irradiation of a body by the radiation of that source comprising a biological protective body (7), a recess made in the biological protective body (7), an orifice (5') discharging on the one hand on the outer wall of the body (7) and on the other on the inside of the said recess in the said body (7), a biological protective disc (8) housed in the recess of the body (7) and adapted for rotary movement, a housing (9) made in the disc (8) and discharging on the periphery of this disc, a radioactive source (10) contained in the housing (9) in the disc (8), means of causing a rotary displacement of the disc (8) between a first position in which the source (10) is opposite the orifice (5') in the body (7) and a second position in which the source (10) is not opposite the orifice (5'), characterised in that the biological protective body (7) comprises a passage (5") discharging at one of its ends on the outer wall of the body (7) and at its other end in the recess in the biological protective body (7), a passage being made in the disc (8), this passage discharging at one and other of its ends in two zones of the lateral wall of the disc (8) adapted to be simultaneously opposite the said orifice (5') and the passage (5"), at least one fibre optic (11) being disposed in the passage in the disc (8), the ends of the fibre (11) referred to respectively as being the receiving end (11a) and the emitting end (11b) being maintained at a right-angle to each of the ends of the passage, an optical device (12) rigid with the body (7) giving rise to a ray of light which falls on the receiving end (11a) of the fibre (11), this device being disposed at a right-angle to the passage (5").

2. Device according to Claim 1, characterised in that it comprises a precollimator (13) disposed at a right-angle to the orifice (5') provided in the biological protective body (7).

3. Device according to Claim 2, characterised in that the axes of the receiving and emitting ends of the fibre (11) define an angle close to 90°.

4. Device according to any one of Claims 1 to 3, characterised in that it comprises electrical means for locking the disc in the said first position.

5. Device according to any one of Claims 1 to 4, characterised in that it comprises mechanical means of automatically placing the disc in the said first position.

6. Device according to any one of Claims 1 to 5, characterised in that the said body (7) is constituted by a lead means (7b) in which there is incorporated a block of impoverished uranium (7a), this block defining the portion of the wall of the cavity situated around the said source when the said disc is in the aforesaid second positon.

7. Device according to any one of Claims 1 to 6, characterised in that the said means of displacing the said disc comprise a shaft (16) coupled on the one hand to a reduction gearing (26) through an electromagnetic clutch (22) and on the other to a rack (32) movable between two extreme positions corresponding respectively to the said first and second positons of the disc (8).

8. Device according to Claim 7, characterised in that the extreme position of the rack (32) corresponding to the said second position of the disc (8) is defined by an electromagnetic stop adapted to lock the rack, this stop acting against a spring (64) tending to restore the rack to an extreme position defined by a mechanical stop.

9. Device according to any one of Claims 1 to 8, characterised in that the enclosure (5) of the irradiation head (3) comprising the precollimator (13) is provided with returned edges which allow it to be temporarily fixed to the movable arm (2) by means of screws.

FIG. 1

FIG. 4b

FIG. 2

0 040 280

FIG. 3

0 040 280

FIG. 4a

FIG. 5

FIG. 6